# FASCICULE DE BREVET EUROPEEN

(11) **EP 3 893 727 B1**
(45) Date de publication et mention de la délivrance du brevet: **31.01.2024**
(21) Numéro de dépôt: 19824202.6
(22) Date de dépôt: 09.12.2019
(51) Int. Cl.: A61B 5/00, A61B 5/053, A61B 5/11, A41D 1/00, A41D 13/06, A61F 13/06, D06F 93/00

(54) **DISPOSITIF DE MESURE DE L'OBSERVANCE THÉRAPEUTIQUE DU PORTE D'UN ARTICLE TEXTILE PAR UN PATIENT, ARTICLE INSTRUMENTE COMPRENANT UN TEL DISPOSITIF, ET MÉTHODE DE MESURE**
VORRICHTUNG ZUR MESSUNG DER THERAPEUTISCHEN EINHALTUNG DES TRAGENS EINES TEXTILARTIKELS DURCH EINEN PATIENTEN, INSTRUMENTIERTER ARTIKEL MIT SOLCH EINER VORRICHTUNG UND MESSVERFAHREN
DEVICE FOR MEASURING THE THERAPEUTIC OBSERVANCE OF THE WEARING OF A TEXTILE ITEM BY A PATIENT, INSTRUMENTED ITEM COMPRISING SUCH A DEVICE, AND MEASUREMENT METHOD

(30) Priorité: 05.12.2018 FR 1872335
(43) Date de publication de la demande: 20.10.2021
(73) Titulaire: Sigvaris AG, 9014 St. Gallen (CH)
(72) Inventeur: GRENIER, Etienne, 42130 Marcilly-le-Chatel (FR); CHAIGNEAU, Cyril, 42000 Saint-Etienne (FR)
(74) Mandataire: Cabinet Laurent & Charras
(86) Numéro de dépôt international: PCT/IB2019/060568
(87) Numéro de publication internationale: WO 2020/115724

(56) Documents cités:
- DE-A1-102008 024 014
- DE-A1-102009 023 228
- FR-A1- 3 051 354
- US-A1- 2017 181 703

## Description

La présente invention concerne un dispositif de mesure de l'observance thérapeutique du porté d'un article textile par un patient. L'invention concerne également un article instrumenté, comprenant un tel dispositif.

Le domaine de l'invention est celui des articles textiles, en particulier les articles de compression et/ou de contention, tels que les chaussettes, bas, collants et manchons, conçus pour équiper les membres inférieurs ou supérieurs d'une personne.

Afin de bénéficier d'un effet thérapeutique optimal, il est essentiel que le porteur de l'article respecte les préconisations du fabricant et du médecin. Différents systèmes ont donc été développés pour évaluer la compliance, c'est-à-dire l'observance du traitement thérapeutique.

Certains systèmes comprennent des étiquettes pour l'appairage des articles, afin de s'assurer que le porteur soit équipé d'une paire d'articles présentant les caractéristiques adaptées à son état de santé.

D'autres systèmes comprennent des capteurs de surface intégrés aux articles, en vue de détecter leur porté.

Cependant, dans la plupart des cas, aucun système n'est intégré à l'article et l'évaluation de l'observance est obtenue par questionnaire d'auto-évaluation réalisé par le patient lui-même. L'inconvénient de cette méthode, même si elle fait référence dans le cadre d'organisation d'études cliniques, est qu'elle entraîne des biais de mesure non négligeables.

WO2014058473 décrit un exemple de dispositif de mesure intégré à un article textile. Le dispositif comprend un substrat flexible, une mémoire, et un capteur capable de mesurer un paramètre d'un utilisateur et/ou de son environnement. Le capteur est étanche, lavable, apte à détecter l'activité électrique et sa position/location. Le capteur peut être constitué par un capteur de capacitance et être utilisé pour détecter la sueur dans les chaussettes. Les données peuvent être transmises par l'intermédiaire d'une antenne NFC formée par une bobine.

US2015145671 décrit un autre exemple de dispositif de mesure, qui présente de nombreuses fonctionnalités, mais est relativement complexe. De préférence, le dispositif comprend un capteur de surface en vue de détecter son porté.

FR 3 051 354 A1 décrit un dispositif de mesure de la durée pendant laquelle une orthèse de compression veineuse élastique est portée, ledit dispositif comportant un capteur capacitif ou une combinaison de capteurs capacitifs.

Le but de la présente invention est de proposer une nouvelle solution de gestion de l'utilisation d'un article de compression et/ou de contention.

A cet effet, l'invention a pour objet un dispositif de mesure de l'observance thérapeutique du porté d'un article textile par un patient, le dispositif comprenant :
- une carte électronique flexible, comportant deux faces opposées, et conçue pour être intégrée à l'article, de sorte que l'une des faces est orientée vers un membre du patient lorsque l'article est porté ;
- deux électrodes capacitives agencées chacune sur l'une des faces de la carte électronique, orientées en sens opposés, et captant des signaux de mesure fonction de leur environnement ;
- un système de contrôle agencé sur la carte électronique et apte à générer des données d'observance à partir des signaux de mesure ; et
- un système de communication agencé sur la carte électronique et conçu pour le transfert de données à distance de l'article ;
le dispositif étant adapté pour détecter sélectivement le porté, le non porté ou le lavage de l'article, ainsi que pour estimer un niveau d'usure potentielle de l'article.

Le dispositif selon l'invention permet de mesurer trois états :
- Lavage si les électrodes mesurent chacune une capacité supérieure à une valeur seuil S1.
- Porté si la différence entre les capacités mesurées par les électrodes est supérieure à une valeur seuil S2.
- Non porté si les capacités mesurées par les électrodes sont inférieures à la valeur seuil S1 et que la différence entre ces capacités est inférieure à la valeur seuil S2.

Ainsi, l'invention permet d'améliorer la mesure de l'observance thérapeutique par le patient utilisant l'article textile. En détectant à la fois le porté, le non-porté et le lavage, le dispositif permet de suivre la compliance du patient à son traitement, mais aussi le respect des recommandations d'utilisation de l'article. En effet, le lavage permet de redynamiser l'effet élastiques des fils de matériau textile et donc l'effet thérapeutique de l'article.

Selon d'autres caractéristiques avantageuses de l'invention, prises isolément ou en combinaison :
- La carte électronique comprend une feuille flexible en matière plastique et un circuit imprimé formé sur la feuille flexible.
- Le dispositif comporte une enveloppe étanche, recouvrant la carte électronique et les éléments solidaires de la carte, y compris les électrodes capacitives.
- L'enveloppe est en matière plastique, notamment en silicone.
- Les électrodes capacitives présentent une sensibilité de mesure fonction de leurs dimensions.
- Les électrodes capacitives comportent un blindage pour diminuer la sensibilité au bruit électrique lié à leur environnement. Le blindage est disposé sous l'électrode, sur l'autre face de la carte électronique.
- A titre d'exemple, une électrode mesure 7 mm de largeur par 7 mm de hauteur, tandis que le blindage mesure 9 mm de largeur par 13 mm de hauteur.
- Les électrodes capacitives sont protégées par un vernis. Le vernis est appliqué sur l'électrode, sur la face de carte électronique correspondante.
- Le système de contrôle comprend un accéléromètre. Les données d'observance sont générées à partir des mesures des électrodes capacitives et de l'accéléromètre.
- Le système de communication comprend une antenne RFID ou NFC.
- Le système de communication est spécifiquement adapté pour communiquer à une fréquence de 13.56 MHz.
- Le système de contrôle et le système de communication sont configurés de sorte que le transfert de données à distance de l'article est réalisable sans utiliser d'énergie embarquée.
- Le système de contrôle génère des données d'observance à partir des signaux de mesure des électrodes capacitives, et les données d'observance sont transférés par le système de communication à distance du dispositif.
- Le système de contrôle génère des données numériques à partir des signaux de mesure des électrodes capacitives, les données numériques sont transférées par le système de communication à distance du dispositif, et les données d'observance sont générées à partir des données numériques hors du dispositif.

L'invention a également pour objet un article instrumenté, comprenant un article textile conçu pour être porté par un patient, et un dispositif tel que mentionné ci-dessus, intégré à l'article.

L'article textile peut être de tout type, tel que chaussette, bas, collant ou manchon, conçu pour équiper un membre inférieur ou supérieur d'une personne.

De préférence, l'article textile est un article de compression et/ou contention.

Le dispositif peut être intégré à l'article textile par tout moyen permettant de garantir son positionnement et son fonctionnement.

Le dispositif peut être inséré entre les deux parois textiles de l'article, par exemple le revers d'une chaussette.

Le dispositif peut être logé dans une poche souple et fine, qui peut être intégrée à l'article par collage, thermocollage et/ou couture.

L'invention a également pour objet une méthode de mesure de l'observance thérapeutique du porté d'un article textile par un patient, mettant en oeuvre un dispositif tel que mentionné ci-dessus. La méthode comprenant les étapes suivantes :
a) une étape de mesure dans laquelle les deux électrodes capacitives orientées en sens opposés captent des signaux de mesure fonction de leur environnement ;
b) une étape de traitement de signaux dans laquelle des données numériques sont générées à partir des signaux de mesure des électrodes capacitives ;
c) une étape de transfert dans laquelle des données sont transférées depuis le dispositif à distance de l'article, ces données comprenant les données d'observance et/ou les données numériques utilisables pour déterminer les données d'observance ;
d) une étape de traitement des données numériques pour générer des données d'observance, soit au sein du dispositif avant l'étape de transfert, soit à distance du dispositif après l'étape de transfert.

### BREVE DESCRIPTION DES DESSINS

L'invention sera mieux comprise à la lecture de la description qui va suivre, donnée uniquement à titre d'exemple non limitatif et faite en référence aux dessins annexés sur lesquels :
- la figure 1 est une vue de côté d'un article instrumenté conforme à l'invention, comprenant un article textile du type chaussette conçu pour être porté par un patient, et un dispositif de mesure d'observance thérapeutique intégré à l'article;
- la figure 2 est une vue de face du dispositif selon l'invention;
- la figure 3 est une vue partielle de dessus du dispositif selon l'invention;
- la figure 4 est un graphe illustrant une mesure de capacité réalisée par une électrode;
- la figure 5 est un tableau illustrant les différents états mesurables par le dispositif; et
- la figure 6 est un graphe illustrant une expérimentation montrant les différents états mesurables par le dispositif.

### EXPOSE DETAILLE DE L'INVENTION

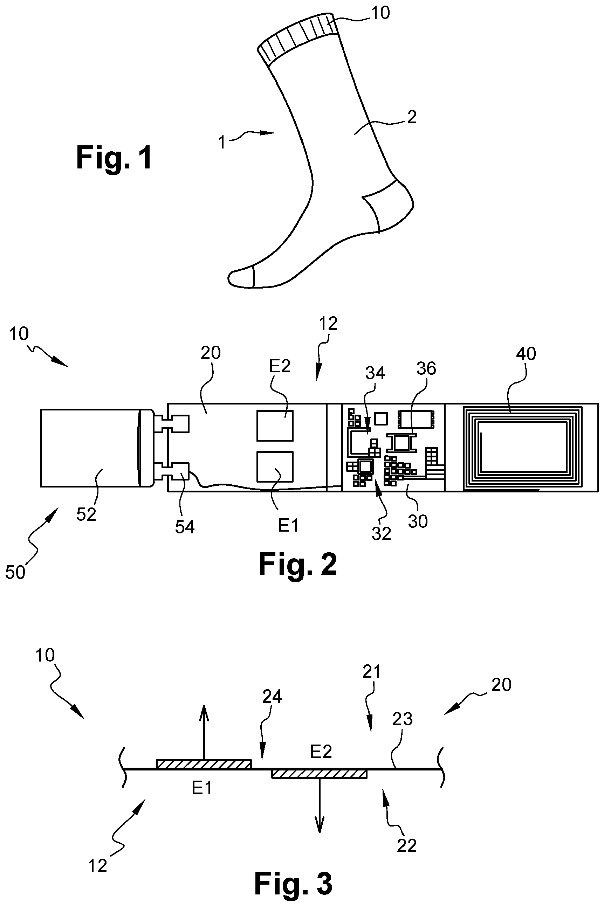

Sur les figures 1 à 3 est représenté un dispositif (10) conforme à l'invention, équipant un article (2) textile du type chaussette de contention. Le dispositif (10) est conçu pour mesurer l'observance thérapeutique du porté de l'article (2) par un patient.

Le dispositif (10) comprend une carte électronique (20), deux électrodes capacitives (E1, E2), un système de contrôle (30), un système de communication (40) et un système d'alimentation (50).

Le dispositif (10) comporte une enveloppe étanche (12), recouvrant intégralement la carte électronique (20) et les éléments (E1, E2, 30, 40, 50) solidaires de la carte (20), y compris les électrodes capacitives (E1, E2). L'enveloppe (12) est de préférence en matière plastique, par exemple en silicone. L'enveloppe (12) peut être transparente, translucide ou opaque.

La carte électronique (20) est mince et comporte deux faces (21, 22) opposées, incluant une face interne (21) et une face externe (22). La carte électronique (20) est flexible, de manière à pouvoir être enroulée autour d'un membre du patient. La carte électronique (20) et conçue pour être intégrée à l'article (2), de sorte que la face interne (21) est orientée vers un membre du patient portant l'article (2). En pratique, le contact entre le dispositif (10) et la peau du patient n'est pas direct, puisqu'il y a une épaisseur de textile et une épaisseur de l'enveloppe étanche (12) entre la peau et la face (21).

La carte électronique (20) comprend une feuille flexible (23) en matière plastique et un circuit imprimé (24) formé sur la feuille flexible (23).

Les deux électrodes capacitives (E1, E2) constituent des capteurs d'observance. Les électrodes (E1, E2) sont conçues pour mesurer une capacité (C), qui varie en fonction de l'environnement. En particulier, la capacité (C) varie en fonction de la proximité avec la peau, l'eau ou l'air.

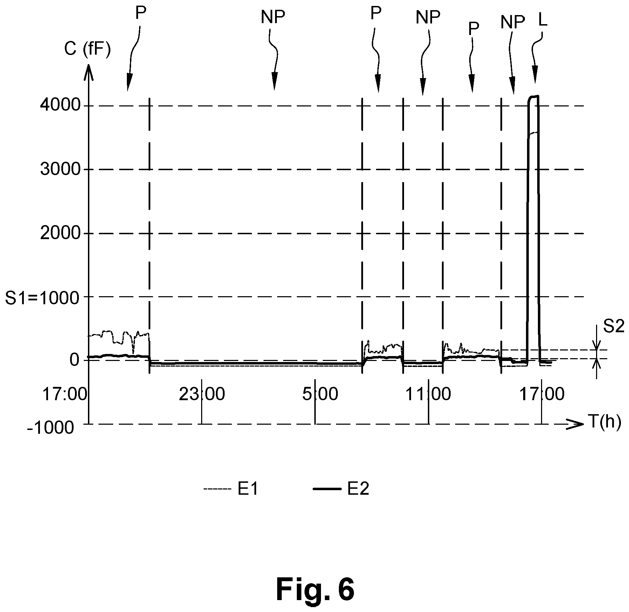

Comme montré sur la figure 6, la capacité (C) est élevée lorsque l'électrode (E1, E2) est à proximité de la peau, très élevée à proximité de l'eau, et très faible à proximité de l'air. La mesure d'observance peut être réalisée directement au contact de la peau, ou à travers une mince épaisseur de tissu.

Les électrodes (E1, E2) peuvent être en cuivre, et formées directement sur le circuit imprimé (24). Les électrodes (E1, E2) sont agencées chacune sur l'une des faces (21, 22) de la carte électronique (20) et orientées en sens opposés. Ainsi, seule une des électrodes (E1, E2) peut être dirigée vers le membre du patient portant l'article (2). Une électrode interne (E1) est agencée sur la face interne (21), et une électrode externe (E2) est agencée sur la face externe (22).

Lorsque la face interne (21) du dispositif (10) est orientée vers un membre du patient portant l'article (2), l'électrode interne (E1) est positionnée contre la peau ou à proximité de la peau du patient.

Le système de contrôle (30) comprend plusieurs composants (32, 34, 36) agencés sur le circuit imprimé (24) de la carte (20).

Le système (30) comprend un microcontrôleur (32) configuré pour traiter les signaux en provenance des électrodes (E1, E2). Plus précisément, le microcontrôleur (32) permet de convertir périodiquement les signaux analogiques issus des électrodes capacitives (E1, E2) en données numériques ou signaux digitalisés. Egalement, le microcontrôleur (32) peut convertir les données numériques ou signaux digitalisés en données d'observance (P / NP / L).

Le système (30) comprend une mémoire (34) configurée pour enregistrer des données numériques après traitement par le microcontrôleur (32).

Le microcontrôleur (32) peut générer les données d'observance (P / NP / L) à partir des signaux issus des électrodes (E1, E2), puis les stocker dans la mémoire (34). En alternative, le microcontrôleur (32) peut générer des données numériques, qui seront traitées après transfert à distance du dispositif (10), pour générer les données d'observance (P / NP / L). Différents modes de fonctionnement du dispositif (30) sont détaillés plus loin.

Le traitement des signaux par le microcontrôleur (32), ainsi que le transfert et l'enregistrement de données dans la mémoire (34), consomment l'énergie du système d'alimentation (50) embarqué par le dispositif (10).

De préférence, le système de contrôle (30) comprend un accéléromètre (36). Ainsi, les données d'observance (P / NP / L) peuvent être générées à partir des mesures des électrodes capacitives (E1, E2) et de l'accéléromètre (36).

En alternative, les données d'observance (P / NP / L) peuvent être générées uniquement à partir des mesures de capacité (C) des électrodes capacitives (E1, E2).

Le système de communication (40) est agencé sur la carte électronique (20) et conçu pour le transfert de données générées par le système de contrôle (30).

Le transfert de données est bidirectionnel : entrant pour la configuration du dispositif (10) et sortant pour la récupération des données par un appareil distant.

Le transfert de données est réalisé par communication radiofréquence avec un lecteur-encodeur, un ordinateur, un téléphone intelligent (smartphone en anglais), ou tout autre système.

De préférence, le système de communication (40) comprend une antenne compatible avec les technologies NFC et/ou RFID. La distance de communication NFC entre l'antenne et un lecteur NFC est de l'ordre de quelques centimètres. La distance de communication RFID entre l'antenne et un lecteur RFID est de l'ordre du mètre.

Encore de préférence, le système (50) communique sur la fréquence 13.58 MHz. La technologie RFID utilise plusieurs bandes de fréquence, dont la fréquence 13.58 MHz. La technologie NFC est basée sur la fréquence 13.58 MHz.

Le système d'alimentation (50) comprend une pile (52) et des connecteurs (54) permettant de connecter la pile (52) à la carte (20). Les connecteurs (54) sont par exemple des plots soudés sur la carte (20). Le système (50) permet d'alimenter le système (30) en énergie.

Avantageusement, la mémoire (34) et le système de communication (40) n'utilisent pas l'énergie embarquée lors de la lecture des données par un appareil distant, tel qu'un smartphone. En effet, l'énergie nécessaire à la mise en fonctionnement de la communication est fournie par l'appareil (lecteur-encodeur), et non par le système (50). Cette énergie peut être apportée par les champs électromagnétiques émis par l'appareil.

Le dispositif (10) est adapté pour déterminer des données d'observance (P / NP / L), c'est à dire détecter sélectivement le porté (P), le non-porté (NP) ou le lavage (L) de l'article (2). Le dispositif (10) permet d'évaluer les durées de porté (P) ou de non-porté (NP), ainsi que le nombre et la fréquence des lavages (L) de l'article (2).

De façon complémentaire, le dispositif (10) permet ainsi d'évaluer le niveau probable d'usure de l'article (2), lié à une durée d'utilisation supérieure à la durée optimale garantie par le fabricant, notamment dans le cas où des données de porté (P) sont encore enregistrées après cette durée optimale. De même, le lavage (L) régulier fait partie des recommandations d'utilisation de l'article (2), car l'action mécanique engendrée permet de redonner à la structure textile ses caractéristiques géométriques initiales. Outre l'aspect hygiénique, un nombre de lavage (L) restreint au regard du temps de porté (P) peut laisser présager d'une efficacité thérapeutique inférieure à celle normalement prévue par le fabricant et retranscrite dans les conditions d'utilisation de l'article (2).

En fonction de sa configuration, le dispositif (10) peut fonctionner suivant l'un des trois modes exposés ci-après.

Par la suite, un slot ("créneau temporel" en français) est défini comme une série de données enregistrées à un instant t.
1er mode de fonctionnement (mode normal) :
   a) Electrodes capacitives (E1, E2) captent des signaux analogiques.
   b) Microcontrôleur (32) transforme signaux analogiques en données numériques.
   c) Microcontrôleur (32) traite données numériques pour générer données d'observance (P / NP / L).
   d) Mémoire (34) enregistre une série de données d'observance (P / NP / L), sans enregistrer les données numériques utilisées pour déterminer les données d'observance (P / NP / L).
   e) Antenne (40) transmet données d'observance (P / NP / L) enregistrées dans la mémoire (34) à un appareil distinct du dispositif (10) via une liaison sans fil.
   Dans ce 1er mode, à chaque slot, seule la donnée d'observance (information porté / non porté / lavage) est enregistrée dans la mémoire (34). Ce mode est relativement économe en énergie et permet de stocker un nombre important de slots. En revanche, il ne permet pas d'avoir accès aux données physiques qui ont été utilisées pour déterminer les données d'observance.
2e mode de fonctionnement (mode log) :
   a) Electrodes capacitives (E1, E2) captent des signaux analogiques.
   b) Microcontrôleur (32) transforme signaux analogiques en données numériques.
   c) Microcontrôleur (32) traite données numériques pour générer données d'observance (P / NP / L).
   d) Mémoire (34) enregistre une série de données numériques et de données d'observance (P / NP / L).
   e) Antenne (40) transmet données numériques et données d'observance (P / NP / L) enregistrées dans la mémoire (34) à un appareil distinct du dispositif (10) via une liaison sans fil.

   Ce 2e mode est plus gourmand en mémoire, mais améliore la traçabilité, en comparaison avec le 1er mode ci-après.
   A titre d'exemple, pour une configuration donnée du dispositif (10), le 1er mode permet d'enregistrer jusqu'à 32704 slots, tandis que le 2e mode permet d'enregistrer jusqu'à 1022 slots.
3e mode de fonctionnement :
   a) Electrodes capacitives (E1, E2) captent signaux analogiques.
   b) Microcontrôleur (32) transforme signaux analogiques en données numériques.
   c) Mémoire (34) enregistre une série de données numériques.
   d) Antenne (40) transmet données numériques enregistrées dans la mémoire (34) à un appareil distinct du dispositif (10) via une liaison sans fil.
   e) Appareil traite données numériques pour générer données d'observance (P / NP / L).
   Ce 3e mode offre un compromis entre les 1er et 2e modes, concernant l'utilisation de la mémoire (34) et de la pile (52). La génération des données d'observance (P / NP / L) est externalisée hors du dispositif (10).

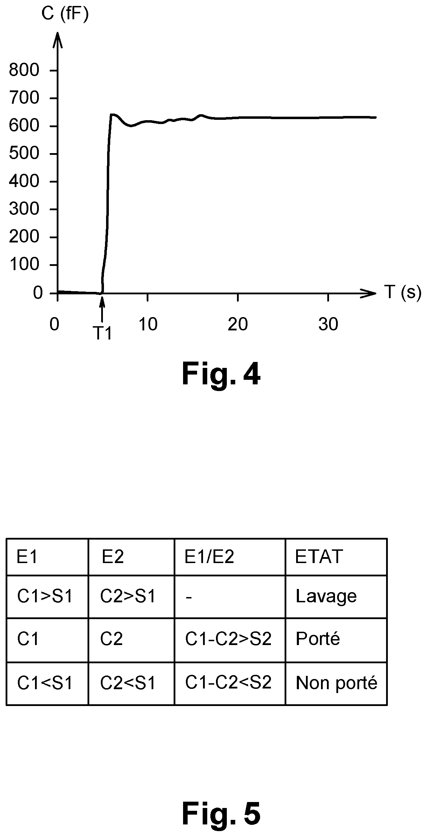

Sur la figure 4 est représenté un graphe montrant une mesure de capacité (C) réalisée par l'une des électrodes (E1, E2). Le temps (T) en secondes (s) est représenté en abscisses, tandis que la capacité (C) en femtofarads (fF) est représentée en ordonnées. La capacité (C) représente la quantité de charge électrique portée par un élément conducteur, pour un potentiel électrique donné.

Sur le graphe, la capacité (C) augmente à un temps (T1) correspondant à 5 secondes environ, lorsque l'électrode est mise en contact avec le membre du patient.

La peau et l'eau ont des propriétés capacitives, très différentes de celles de l'air. La capacité (C) issue de chaque électrode (E1, E2) varie en fonction d'un contact ou d'une proximité avec la peau ou l'eau.

Sur la figure 5 est représenté un tableau montrant les différents états mesurables par le dispositif (10) :
- Lavage (L) si les deux électrodes (E1, E2) mesurent chacune une capacité (C) supérieure à une valeur seuil (S1). Cette valeur seuil (S1) est paramétrable et fournie au système (30) lors de sa configuration. La capacité (C) mesurée par l'électrode externe (E2) est généralement supérieure à la capacité (C) mesurée par l'électrode interne (E1). En complément, l'accéléromètre (36) permet de détecter les mouvements particuliers de l'article (2) disposé dans une machine à laver.
- Porté (P) si la différence entre les capacités (C) mesurées par les deux électrodes (E1, E2) est supérieure à une valeur seuil (S2). Cette valeur seuil (S2) est paramétrable et fournie au système (30) lors de sa configuration. La capacité (C) mesurée par l'électrode interne (E1) est supérieure à la capacité (C) mesurée par l'électrode externe (E2). En complément, l'accéléromètre (36) permet de détecter les mouvements du patient.
- Non porté (NP) si les capacités (C) mesurées par les deux électrodes (E1, E2) sont inférieures à la valeur seuil (S1) et que la différence entre ces capacités (C) est inférieure à la valeur seuil (S2). En complément, l'accéléromètre (36) permet de détecter l'immobilité de l'article (2).

Sur la figure 6 est représenté un graphe similaire à celui de la figure 6, montrant les mesures de capacité (C) réalisées par les deux électrodes (E1, E2). Le temps (T) en heures (h) est représenté en abscisses, tandis que la capacité (C) en femtofarads (fF) est représentée en ordonnées.

Le graphe montre une succession d'états porté (P) et non-porté (NP), puis un lavage (L) de l'article (2). Lors du porté (P), on remarque l'écart supérieur à la valeur seuil (S2) entre les mesures de capacité (C) des deux électrodes (E1, E2). Lors du non-porté (NP), on remarque la similarité entre les mesures de capacité (C) des deux électrodes (E1, E2), à une valeur d'étalonnage proche de zéro. Lors du lavage (L), on remarque les mesures de capacité (C) très élevées des deux électrodes (E1, E2), bien supérieures à la valeur seuil (S1).

Si un état change sur une courte durée, sa valeur est remplacée par l'état voisin (moyennage des états au court du temps). C'est par exemple le cas lorsque l'article (2) est porté (P), et que la différence entre les capacités (C) mesurées par les deux électrodes (E1, E2) descend brièvement en dessous de la valeur seuil (S2).

L'article (2) et le dispositif (10) peuvent être conformés différemment des figures 1 à 3 sans sortir du cadre de l'invention, définie par les revendications. En outre, les caractéristiques techniques des différents modes de réalisation et variantes mentionnés ci-dessus peuvent être, en totalité ou pour certaines d'entre elles, combinées entre elles. Ainsi, le dispositif (10) peut être adapté en termes de coût, de fonctionnalités et de performance.

## Revendications

1. Dispositif (10) de mesure de l'observance thérapeutique du porté d'un article textile (2) par un patient, le dispositif (10) comprenant :
- une carte électronique (20) flexible, comportant deux faces (21, 22) opposées, et conçue pour être intégrée à l'article (2), de sorte que l'une des faces (21, 22) est orientée vers un membre du patient lorsque l'article (2) est porté ;
- deux électrodes capacitives (E1, E2) captant des signaux de mesure fonction de leur environnement ;
- un système de contrôle (30) agencé sur la carte électronique (20) et apte à générer des données d'observance (P / NP / L) à partir des signaux de mesure ; et
- un système de communication (40) agencé sur la carte électronique (20) et conçu pour le transfert de données à distance de l'article (2) ;
le dispositif (10) étant adapté pour détecter sélectivement le porté, le non-porté ou le lavage de l'article (2), ainsi que pour estimer un niveau d'usure potentielle de l'article (2) ;
**caractérisé en ce que** les électrodes capacitives (E1, E2) sont agencées chacune sur l'une des faces (21, 22) de la carte électronique (20) et orientées en sens opposés.

2. Dispositif (10) selon la revendication précédente, **caractérisé en ce que** la carte électronique (20) comprend une feuille flexible (23) en matière plastique et un circuit imprimé (24) formé sur la feuille flexible (23).

3. Dispositif (10) selon l'une quelconque des revendications précédentes, **caractérisé en ce que** le dispositif (10) comporte une enveloppe étanche (12).

4. Dispositif (10) selon l'une quelconque des revendications précédentes, **caractérisé en ce que** le système de contrôle (30) comprend un accéléromètre (36), et **en ce que** les données d'observance (P / NP / L) sont générées à partir des mesures des électrodes capacitives (E1, E2) et de l'accéléromètre (36).

5. Dispositif (10) selon l'une quelconque des revendications précédentes, **caractérisé en ce que** le système de contrôle (30) et le système de communication (40) sont configurés de sorte que le transfert de données à distance de l'article (2) est réalisable sans utiliser d'énergie embarquée.

6. Dispositif (10) selon l'une quelconque des revendications 1 à 5, **caractérisé en ce que** le système de contrôle (30) génère des données d'observance (P / NP / L) à partir des signaux de mesure des électrodes capacitives (E1, E2), et les données d'observance (P / NP / L) sont transférés par le système de communication (40) à distance du dispositif (10).

7. Dispositif (10) selon l'une quelconque des revendications 1 à 5, **caractérisé en ce que** le système de contrôle (30) génère des données numériques à partir des signaux de mesure des électrodes capacitives (E1, E2), les données numériques sont transférées par le système de communication (40) à distance du dispositif (10), et les données d'observance (P / NP / L) sont générées à partir des données numériques hors du dispositif (10).

8. Article instrumenté (1), comprenant un article textile (2) conçu pour être porté par un patient, et un dispositif (10) selon l'une des revendications 1 à 7, intégré à l'article (2).

9. Article instrumenté (1) selon la revendication 8, **caractérisé en ce que** l'article (2) est un article de compression et/ou contention.

10. Méthode de mesure de l'observance thérapeutique du porté d'un article textile (2) par un patient, mettant en oeuvre un dispositif (10) selon l'une des revendications 1 à 7, la méthode comprenant les étapes suivantes :
a) une étape de mesure dans laquelle les deux électrodes capacitives (E1, E2) orientées en sens opposés captent des signaux de mesure fonction de leur environnement ;
b) une étape de traitement de signaux dans laquelle des données numériques sont générées à partir des signaux de mesure des électrodes capacitives (E1, E2) ;
c) une étape de transfert dans laquelle des données sont transférées depuis le dispositif (10) à distance de l'article (2) ;
d) une étape de traitement des données numériques pour générer les données d'observance (P / NP / L), soit au sein du dispositif (10) avant l'étape de transfert, soit à distance du dispositif (10) après l'étape de transfert.

## Patentansprüche

1. Messvorrichtung (10) für die Therapietreue beim Tragen eines textilen Artikels (2) durch einen Patienten, dabei umfasst die Vorrichtung (10):
- eine flexible Speicherkarte (20) mit zwei entgegengesetzten Seiten (21, 22), und dazu vorgesehen, in den Artikel (2) integriert zu werden, so dass eine der Seiten (21, 22) zu einem Glied des Patienten hin orientiert ist, wenn der Artikel (2) getragen wird ;
- zwei kapazitive Elektroden (E1, E2) zum Auffangen von umgebungsabhängigen Messsignalen;
- ein Kontrollsystem (30), angeordnet auf der Speicherkarte (20), das in der Lage ist Compliance- Daten (P / NP / L) ausgehend von Messsignalen zu erzeugen; und
- ein Kommunikationssystem (40), angeordnet auf der Speicherkarte (20) und vorgesehen für die Fernübertragung von Daten des Artikels (2),
die Vorrichtung (10) eignet sich zur selektiven Erkennung des Tragens, des Nicht-Tragens oder des Waschens des Artikels (2), sowie zur Einschätzung eines potentiellen Abnutzungsniveaus des Artikels (2),
**dadurch gekennzeichnet, dass** die kapazitiven Elektroden (El, E2) jeweils auf einer der Seiten (21, 22) der Speicherkarte (20) angeordnet sind und in die Gegenrichtung ausgerichtet sind.

2. Vorrichtung (10) nach dem vorangehenden Anspruch, **dadurch gekennzeichnet, dass** die Speicherkarte (20) eine flexible Folie (23) aus Kunststoff sowie eine Platine (24) enthält, die auf dieser flexiblen Folie (23) gebildet ist.

3. Vorrichtung (10) nach irgendeinem der vorstehenden Ansprüche, **dadurch gekennzeichnet, dass** die Vorrichtung (10) eine dichte Hülle (12) enthält.

4. Vorrichtung (10) nach irgendeinem der vorstehenden Ansprüche, **dadurch gekennzeichnet, dass** das Kontrollsystem (30) einen Beschleunigungsmesser (36) enthält, und dass die Compliance- Daten (P / NP / L) ausgehend von den Messungen der kapazitiven Elektroden (E1, E2) und des Beschleunigungsmessers (36) erzeugt werden.

5. Vorrichtung (10) nach irgendeinem der vorstehenden Ansprüche, **dadurch gekennzeichnet, dass** das Kontrollsystem (30) und das Kommunikationssystem (40) so konfiguriert sind, dass die Femdatenübertragung des Artikels (2) ohne Batterie durchführbar ist.

6. Vorrichtung (10) nach irgendeinem der vorstehenden Ansprüche 1 bis 5, **dadurch gekennzeichnet, dass** das Kontrollsystem (30) Compliance- Daten (P / NP / L) ausgehend von den Messsignalen der kapazitiven Elektroden (E1, E2) erzeugt und die Compliance- Daten (P / NP / L) über das Kommunikationssystem (40) zur Femdatenübertragung der Vorrichtung (10) übertragen werden.

7. Vorrichtung (10) nach irgendeinem der vorstehenden Ansprüche 1 bis 5, **dadurch gekennzeichnet, dass** das Kontrollsystem (30) numerische Daten, ausgehend von den Messsignalen der kapazitiven Elektroden (E1, E2) erzeugt, die numerischen Daten werden über das Kommunikationssystem (40) zur Femdatenübertragung der Vorrichtung (10) übertragen, und die Compliance- Daten (P / NP / L) werden ausgehend von numerischen Daten außerhalb der Vorrichtung (10) erzeugt.

8. Instrumentierter Artikel (1), der einen textilen Artikel (2) umfasst, der dazu entwickelt wurde, von einem Patienten getragen werden sowie eine in den Artikel (2) integrierte Vorrichtung (10) nach einem der Ansprüche 1 bis 7.

9. Instrumentierter Artikel (1) nach Anspruch 8, **dadurch gekennzeichnet, dass** der Artikel (2) ein Artikel zur Kompression und/ oder Ruhigstellung ist.

10. Messverfahren für die Therapietreue beim Tragen eines textilen Artikels (2) durch einen Patienten, bei dem eine Vorrichtung (10) nach einem der Ansprüche 1 bis 7 eingesetzt wird, dabei umfasst das Verfahren die folgenden Schritte:
a) einen Schritt der Messung, in dem die beiden in entgegengesetzte Richtungen ausgerichteten kapazitiven Elektroden (E1, E2) Messsignale erfassen, die von ihrer Umgebung abhängen;
b) einen Schritt der Signalverarbeitung, bei der numerische Daten ausgehend von Messsignalen der kapazitiven Elektroden (E1.E2) erzeugt werden:
c) einen Schritt der Übertragung, in dem die Daten von der Fernübertragungsvorrichtung (10) des Artikels (2) übertragen werden,
d) einen Schritt der numerischen Daten zur Erzeugung der Compliance- Daten (P / NP / L), entweder innerhalb der Vorrichtung (10), vor dem Schritt der Übertragung oder entfernt von der Vorrichtung (10), nach dem Schritt der Übertragung.

## Claims

1. A device (10) for measuring therapeutic compliance with wearing a textile item (2) by a patient, the device (10) comprising:
- a flexible electronic card (20) comprising two opposed faces (21, 22) and designed to be integrated into the item (2) in a manner such that one of the faces (21, 22) is orientated towards a limb of the patient when the item (2) is worn;
- two capacitive electrodes (E1, E2) capturing measuring signals which are a function of their environment;
- a monitoring system (30) disposed on the electronic card (20) and capable of generating compliance data (P/NP/L) from the measuring signals; and
- a communications system (40) disposed on the electronic card (20) and designed to transfer data remotely from the item (2);
the device (10) being suitable for selectively detecting Wearing, Not Wearing or Washing of the item (2), as well as for estimating a potential level of wear of the item (2), **characterized in that** the capacitive electrodes (E1, E2) are each disposed on one of the faces (21, 22) of the electronic card (20), orientated in opposing directions.

2. The device (10) as claimed in the preceding claim, **characterized in that** the electronic card (20) comprises a flexible foil (23) of plastic material and a printed circuit (24) formed on the flexible foil (23).

3. The device (10) as claimed in any one of the preceding claims, **characterized in that** the device (10) comprises an impervious envelope (12).

4. The device (10) as claimed in any one of the preceding claims, **characterized in that** the monitoring system (30) comprises an accelerometer (36) and **in that** the compliance data (P/NP/L) are generated from measurements from the capacitive electrodes (E1, E2) and from the accelerometer (36).

5. The device (10) as claimed in any one of the preceding claims, **characterized in that** the monitoring system (30) and the communications system (40) are configured in a manner such that the transfer of data remotely from the item (2) can be carried out without the use of onboard energy.

6. The device (10) as claimed in any one of claims 1 to 5, **characterized in that** the monitoring system (30) generates compliance data (P/NP/L) from the measuring signals from the capacitive electrodes (E1, E2), and the compliance data (P/NP/L) are transferred by the communications system (40) remotely from the device (10).

7. The device (10) as claimed in any one of claims 1 to 5, **characterized in that** the monitoring system (30) generates digital data from the measuring signals from the capacitive electrodes (E1, E2), the digital data are transferred by the communications system (40) remotely from the device (10), and the compliance data (P/NP/L) are generated from digital data exclusively of the device (10).

8. An instrumented item (1) comprising a textile item (2) designed to be worn by a patient, and a device (10) as claimed in one of claims 1 to 7, integrated into the item (2).

9. The instrumented item (1) as claimed in claim 8, **characterized in that** the item (2) is a compression and/or support item.

10. A method for measuring therapeutic compliance with wearing a textile item (2) by a patient, using the device (10) as claimed in one of claims 1 to 7, the method comprising the following steps:
a) a measuring step, in which the two capacitive electrodes (E1, E2) orientated in opposing directions capture measuring signals which are a function of their environment;
b) a signal processing step, in which digital data are generated from the measuring signals from the capacitive electrodes (E1, E2);
c) a transfer step, in which data are transferred from the device (10) remotely from the item (2);
d) a digital data processing step, in order to generate the compliance data (P/NP/L), either within the device (10) before the transfer step, or remotely from the device (10) after the transfer step.
